# EUROPÄISCHE PATENTSCHRIFT

(11) **EP 0 646 593 B1**
(45) Veröffentlichungstag und Bekanntmachung des Hinweises auf die Patenterteilung: **05.12.2001**
(21) Anmeldenummer: 94115348.8
(22) Anmeldetag: 29.09.1994
(51) Int. Cl.: C07J 5/00, A61K 31/56, A61K 31/58, C07J 41/00, C07J 31/00, C07J 43/00, C07J 33/00, C07J 17/00

(54) **Corticoid-17,21-dicarbonsäureester sowie Verfahren zu deren Herstellung und diese enthaltende Arzneimittel**
Corticoid-17,21-dicarboxylic acid ester, process for their production and medicaments containing them
Ester d'acide corticoide-17,21-dicarboxylique, procédé pour leur préparation et médicaments les contenant

(30) Priorität: 05.10.1993 DE 4333920
(43) Veröffentlichungstag der Anmeldung: 05.04.1995
(73) Patentinhaber: HOECHST AKTIENGESELLSCHAFT, 65929 Frankfurt am Main (DE)
(72) Erfinder: Stache, Ulrich, Dr., D-65719 Hofheim (DE); Alpermann, Hans-Georg, Dr., D-61462 Königstein (DE); Dürckheimer, Walter, Dr., D-65795 Hattersheim (DE); Bohn, Manfred, Dr., D-65719 Hofheim (DE)

(56) Entgegenhaltungen:
- EP-A- 0 000 742
- EP-A- 0 072 200
- EP-A- 0 156 643
- THE JOURNAL OF STEROID BIOCHEMISTRY AND MOLECULAR BIOLOGY, Bd.44, Nr.2, Februar 1993, OXFORD, GB Seiten 141 - 145 M. ISOGAI ET AL 'Binding affinities of mometasone furoate and related compounds including its metabolites for the glucocorticoid receptor of rat skin tissue.'

## Beschreibung

### Die Erfindung betrifft Corticoid-17,21-dicarbonsäureester der Formel I

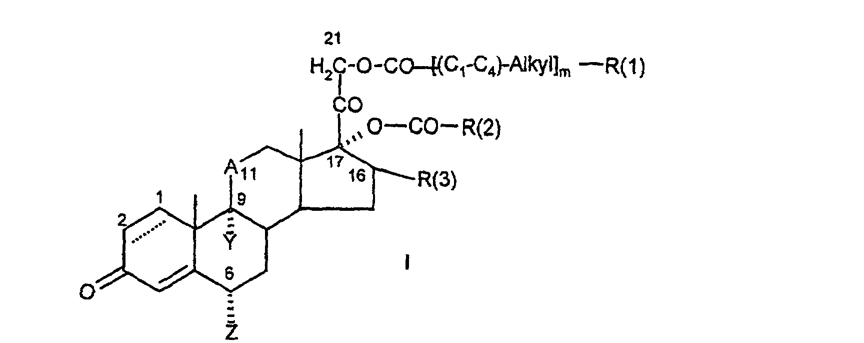

in welcher bedeuten:
- A: CHOH und CHCI in beliebiger sterischer Anordnung, (CH₂), C=O, 9(11)-Doppelbindung;
- Y: Wasserstoff, Fluor, Chlor;
- Z: Wasserstoff, Fluor, Methyl;
- R(1): Phenyl, das unsubstituiert ist oder substituiert mit Methylendioxy, Halogen, Alkyl, Alkoxy, NO₂, NH₂ oder CN;
[(C₁-C₄)-Alkyl]
gesättigt, geradkettig oder durch weitere Alkylgruppen verzweigt;
- m: 1;
- R(2): Phenyl oder Benzyl;
- R(3): Wasserstoff oder α- oder β-Methyl.

Bevorzugt sind die Verbindungen der Formel I Prednisolon-17-benzoat-21-phenylacetat oder um Betamethason-17-benzoat-21-phenylacetat.

Die Erfindung betrifft auch ein Verfahren zum Herstellen einer Verbindung I nach Anspruch 1, dadurch gekennzeichnet, daß man
a) eine Verbindung der Formel II, 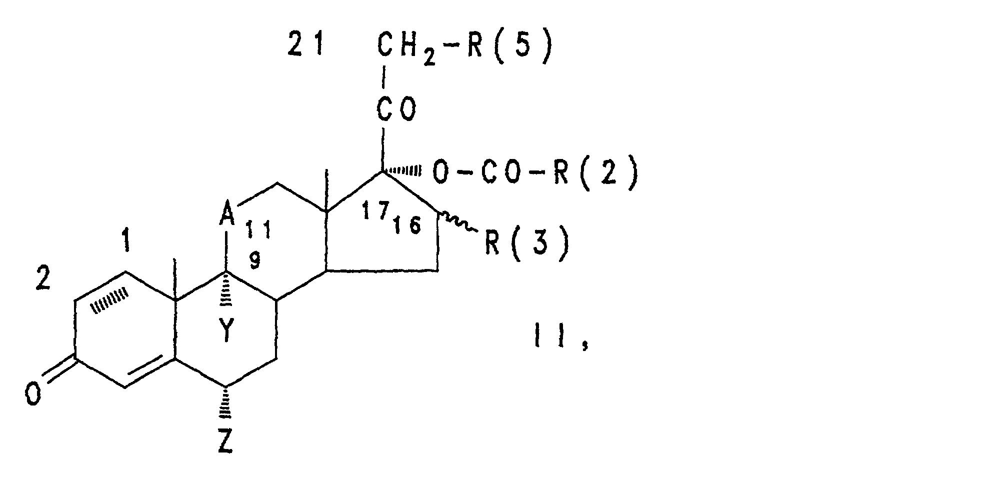 in der R(5) gleich OH ist und die übrigen Substituenten die in Anspruch 1 angegebenen Bedeutungen haben,
a 1) mit einer aktivierten Carbonsäure der Formel III, vorzugsweise einem Halogenid oder Anhydrid oder Azolid,

   R(6)-CO-[(C₁-C₄)-Alkyl]ₘ-R(1) III

   umsetzt, wobei bedeuten:
   - m: 1, und
   [(C₁-C₄)-Alkyl] und R(1) die in Anspruch 1 angegebenen Bedeutungen haben und
   - R(6): Cl, Br, O[-CO-(O)ₙ-[(C₁-C₄)-Alkyl]ₘ-R(1)]₁-, -O-C(O)-CF₃ oder ein anderes aktiviertes Säureradikal, oder
a 2) mit einer Carbonsäure der Formel III selbst, in der
   R(6) OH ist,
   und die weiteren Substituenten bei Formel lll angegeben sind,
   in Gegenwart Wasser abspaltender Reagentien (DCCI etc.) umsetzt
   oder daß man
b) Verbindungen der Formel II, 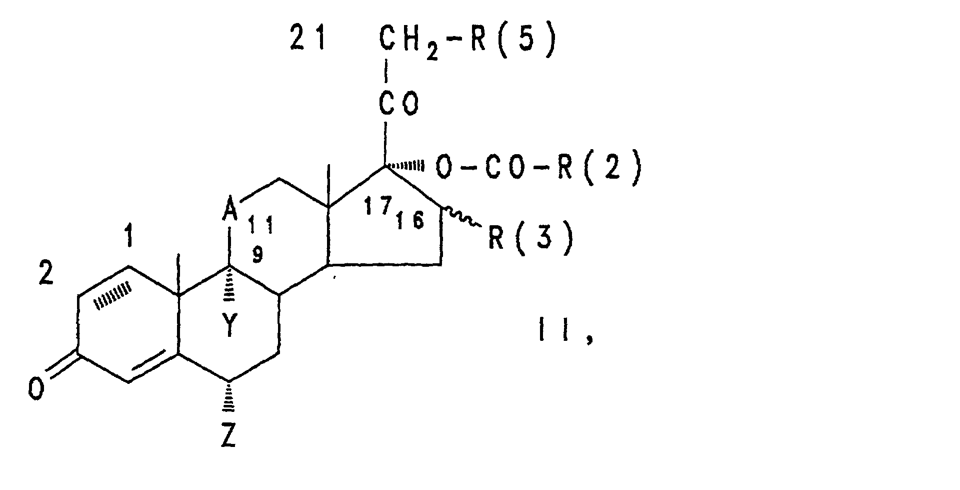 in der R(5) = Br, J, eine Sulfonsäurearyl- oder -alkylestergruppierung ist und die weiteren Substituenten die bei Formel I angegebenen Bedeutung haben, mit einem Salz, vorzugsweise K- oder Na-Salz oder einem Trialkylammoniumsalz, einer Carbonsäure der Formel III,

   R(6)-CO- [(C₁-C₄)-Alkyl]ₘ-R(1) III

   in der
   R(6) -[O⁻Me⁺] bedeutet,
   und die weiteren Substituenten die bei Formel III angegebenen Bedeutungen
   haben,
   umsetzt,
   wobei Me vorzugsweise das Kation eines Alkalisalzes oder eines Trialkylammoniumsalzes ist.

Aus der EP 72 200 sind verwandte Corticoide bekannt; sie nimmt aber die spezielle Substitution in 21- Stellung, nämlich die Phenylacetete, weder vorweg, noch legt sie diese nahe. Sie befasst sich nur mit aliphatischen Substituenten in Stellung 21.

Die als Ausgangssubstanzen benötigten Steroid-17-carbonsäureester mit freier 21-Hydroxylgruppe der Formel II [R(5) = OH] sind in der Regel bekannt oder werden nach bekannten Verfahren hergestellt.

Die punktierte Linie zwischen den C-Atomen 1 und 2 zeigt an, daß diese Bindung eine Einfachbindung oder eine ungesättigte Bindung sein kann.

Die Steroid-17-carbonsäureester mit R(5) gleich Br, J, -OSO₂-Aryl, -OSO₂-Alkyl in Formel II sind in der Regel bekannt oder werden nach bekannten Verfahren hergestellt, z. B. in Analogie zu entsprechenden Corticoid-17-alkylcarbonat-21-Verbindungen nach der US-Patentschrift 4 377 575 und der europäischen Offenlegungsschrift 470 617. Hierbei kommen die 17-Carbonsäureester folgender Costicosteroide in Frage:

Prednisolon, Prednison, 6α-Methyl-prednisolon, 6α,16 α-Dimethylprednisolon, 16 α -Methyl-prednisolon, Hydrocortison (Cortisol), Cortison, 6α -Methyl-cortisol, Reichsteins Substanz S, 11-Desoxi-9(11)-dehydro-prednisolon, 6α-Fluorprednisolon, Dexamethason, 6α-Fluor-dexamethason, 9α-Fluorprednisolon, 6α,9α-Difluor-prednisolon, 6α-Methyl,9α fluor-prednisolon, Betamethason, Clobetasol.

Die als Reaktionspartner zum Einsatz kommenden Carbonsäuren der Formel III [R(6) gleich OH] bzw. deren aktivierte Derivate, wie die Halogenide [R(6) = Cl, Br, J oder deren Anhydride], oder deren Azolide [R(6) gleich Imidazolid, Triazolid] oder deren Salze [R(6) gleich (MeO)-, vorzugsweise (KO)-, (NaO)-] sind in der Regel bekannt und werden gegebenenfalls nach allgemeinen präparativen Methoden hergestellt. Beispiele der gemäß der Erfindung zum Einsatz gelangenden Carbonsäuren gemäß Formel III [R(6) gleich OH] findet man in der Liste am Ende des Textes vor den Ansprüchen.

Alle hierunter fallenden Carbonsäuren tragen in ihrem Säurerest eine gegebenenfalls durch Methylendioxy, Halogen, Alkyl, Alkoxyl, Nitro, Amin, oder Cyan substituierte Phenyl-Gruppe. Letztere sind essentieller Bestandteil der Erfindung.

Wie im pharmakologischen Teil gezeigt wird, zeigen insbesondere Corticoid-17,21-dicarbonsäureester dieses Typs (= 21-Aryl- ester-Typ) im Vergleich zu strukturverwandten Corticoid-17,21-dicarbonsäureestern bzw. strukturverwandten Corticoid-17-alkylcarbonat-21-carbonsäureestern, die keine Aryl-gruppe im 21-Säurerest tragen, oft deutlich bessere Wirkqualitäten hinsichtlich des Verhältnisses lokale/systemische antiinflammatorische Wirkung.

Detaillierte Beschreibung der einzelnen Reaktionsführungen der Herstellungsverfahren für die erfindungsgemäßen Verfahrensprodukte gemäß Formel 1:

### zu Verfahrensvariante a:

Zur Herstellung von 21-Carbonsäureestem des o. a. Typs werden vorzugsweise entweder Carbonsäurehalogenide oder-azolide der Formel III

R(6)-OC-[(C₁-C₄-Alkyl]ₘ-R(1) III,

in der bedeuten: 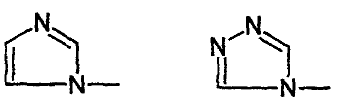
- R(6): Cl, Br, J,
- m: 1 und
R(1) sowie [(C₁-C₄)-Alkyl] die zur Formel III angegebenen Bedeutungen haben
oder Carbonsäureanhydride der Fonnel V

O{-OC-[(C₁-C₄)-Alkyl]ₘ-R(1)}₂ V,

in der bedeuten:
- m: 1, und
R(1) sowie [(C₁-C₄)- Alkyl] die zur Formel III angegebenen Bedeutungen haben, verwendet. In beiden Fällen können die ihnen zugrundeliegenden in der Liste aufgeführten Carbonsäuren verwendet werden, vorzugsweise deren Carbonsäurechloride, -anhydride und -imidazolide bzw. -triazolide.

R(6) in Formel III können auch andere die Carboxylgruppe in Carbonsäuren die Veresterung aktivierende Gruppen beinhalten, so beispielsweise -O-CO-CF₃ oder die aus Phosphon- oder Phosphinsäureanhydriden (z. B. Propanphosphonsäureanhydrid) oder Polyphosphorsäureanhydrid (PPA) herstellbaren aktivierten Carbonsäuren.
Weitere Phosphorreagentien, die eine schonende Veresterung von organischen Carbonsäuren mit der 21-Alkoholgruppe von Corticoid-17-alkylcarbonaten bewirken können, sind in den Literaturstellen Synth. Commun. 13, 471ff(1983) und Synth. Commun. 14, 515ff (1984) angeführt bzw. beschrieben.

Zur Veresterung mit einem Carbonsäurehalogenid oder -anhydrid oder einem Halogenformat löst man die Steroidkomponente in einem inerten Lösungsmittel, beispielsweise in einem Ether, wie Dioxan, Tetrahydrofuran, Diglym, oder gegebenenfalls halogenierten Kohlenwasserstoffen, wie Benzol, Toluol, Cyclohexan, Methylenchiorid, Chloroform oder in Aceton oder in einem Gemisch dieser Lösungsmittel. Zur Entfernung der in der Reaktion entstehenden Halogenwasserstoffsäure setzt man 1 bis 1000 Moläquivalente einer tertiären Base, wie Pyridin, Chinolin, Triethylamin, Dimethylanilin, Dimethylaminopyridin usw., zu. Man kann aber auch eine anorganische Base, wie Natriumhydrogencarbonat oder Calciumcarbonat, zur Entfernung der Säure benutzen. Anschließend tropft man 1 bis 200 Moläquivalente, vorzugsweise 1 bis 3 Moläquivalente eines der oben angeführten Acylierungsmittel, gegebenenfalls gelöst in einem der oben angeführten Lösungsmittel, bei einer Temperatur von -40°C bis zum Siedepunkt des verwendeten Lösungsmittels, vorzugsweise von 0 bis 25°C, zu. Anschließend läßt man das Reaktionsgemisch eine bis 120 Stunden bei einer Temperatur von -40°C bis zum Siedepunkt des Lösungsmittels, vorzugsweise von 0 bis 25°C stehen.

Bei Verwendung von Carbonsäureanhydriden als Acyrierungsmittel ist es hin und wieder von Vorteil, ohne Zusatz von Lösungsmitteln zu arbeiten. Es reicht in der Regel aus, lediglich die organische Base, vorzugsweise Pyridin, dem gegebenenfalls im Überschuß angewandten Säureanhydrid zuzufügen.

Insbesondere bei empfindlichen (und zuweilen instabilen) Carbonsäurederivaten des o. a. Typs, insbesondere bei Verwendung von Phenylacetylchloriden und - anhydriden ist es von großem präparativem und reaktionsselektivem Vorteil, die Corticoid-17-carbonsäureester mit freier 21-Hydroxylgruppe mit 1 bis 4 Moläquivalenten des Chlorids bzw. Anhydrids bei -10 bis +6°C (maximal 20°C) in chlorierten Kohlenwasserstoffen, wie vorzugsweise Dichlormethan, sowie mit 1 bis 4 Moläquivalenten einer Pyridinbase, vorzugsweise Dimethylaminopyridin, umzusetzen.

Hierbei werden die Reaktionsprodukte der Formel I in hoher Reinheit, ohne nennenswerte Beimengungen an Nebenprodukten, insbesondere 11-acylierten Produkten, erhalten (Verfolgung der Reaktionsführungen durch DC), das heißt die Reaktionsführungen sind hinsichtlich der Umsetzung der 21-Hydroxygruppe hoch regioselektiv.

Bei den Reaktionen mit Carbonsäurechloriden wird in vorteilhafter Weise oft absolutes Dioxan oder Tetrahydrofuran zum Reaktionsgemisch gegeben, z. B. bei Benzoylchlorid, wobei z. B. das Verhältnis Dioxan/Pyridin etwa 1:1 ist, und zur Reaktionsbeschleunigung wird das Reaktionsgemisch oft, insbesondere bei sterisch gehinderten oder weniger reaktiven Carbonsäurechloriden oder -anhydriden auf etwa 60°C erwärmt (DC-Verfolgung der Reaktionsverläufe).

Die Charakterisierung der Reaktionsprodukte kann durch Dünnschicht-Chromatographie (DC) erfolgen; hierbei haben die Reaktionsprodukte R_{F}-Werte von etwa 0,65 bis 0,8. In der Regel werden die Reaktionsprodukte durch Massenspektren mit MS = m/z = .... (M+H⁺) charakterisiert (in der Regel FAB-Spektren); es werden jeweils die monoisotopischen Molmassen erfaßt. Die M+H⁺-Werte wurden jeweils aufgerundet. Auch IR-, ¹H-NMR-und UV-Spektren können zur Charakterisierung herangezogen werden.

Zur Aufarbeitung gießt man das Reaktionsgemisch in Wasser, das gegebenenfalls mit Natriumchlorid und Natriumbicarbonat versetzt wurde, wobei die Reaktionsprodukte, oft erst nach längerem Stehen, im allgemeinen kristallin ausfallen. Ölig oder wachsartig gebliebene Reaktionsprodukte werden durch Ausschütteln mit einem geeigneten Extraktionsmittel und Eindampfen angereichert. Die Reaktionsprodukte können, falls erforderlich, durch Umkristallisieren oder durch Chromatographie aufgetrennt oder gereinigt werden. Oft genügt auch intensives Digerieren in einem das Reaktionsprodukt möglichst wenig oder nicht lösenden organischen Lösungsmittel, wie Diethylether oder Cyclohexan, oder einem Gemisch aus diesen Komponenten zur weiteren Reinigung der Reaktionsprodukte.

Bei Verwendung von Carbonsäureazoliden führt man die Veresterung zweckmäßig als Eintopfreaktion durch. Hierbei löst man beispielsweise Aryl-essigsäure oder eine andere Carbonsäure der Formel III [R(6) gleich OH), n gleich Null], in absolutem Pyridin und gibt eine vorzugsweise äquimolare Menge N,N-Carbonyl-diimidazol oder -[1H-1,2,4-triazol] hinzu, wobei sich bei 0 bis 20°C die entsprechenden Säureazolide bilden. Nach Zugabe einer etwa äquimolaren Menge Corticoid-17-carbonsäureester der Formel II [R(5) = OH] und katalytischer Menge einer Base, vorzugsweise Natriumhydrid oder -imidazotid rührt man in Pyridin zwischen 0 bis 40°C; vorzugsweise 20°C und arbeitet wie üblich auf.

Man kann aber auch das vorher durch äquimolare Mengen N,N'-Carbonylazolid und Carbonsäure in absolutem Tetrahydrofuran hergestellte und isolierte Carbonsäureazolid in Lösungsmitteln wie Pyridin, Dimethylformamid, Tetrahydrofuran im gelösten Steroid zugeben und weiter wie oben geschildert verfahren [s. auch Chem. Ber. 95, S. 1284 ff. (1962)].

Bei der Veresterung mit Hilfe von Phosphon- bzw. Phosphinsäureanhydriden setzt man vorzugsweise äquimolare Mengen Carbonsäure und Corticoid-21-alkohol in absolutem Pyridin mit 50 %igen Propanphosphorsäureanhydrid in Methylenchlorid bei 20 bis 60°C unter Zugabe von 4-Dimethylaminopyridin als Säurefänger hinzu und arbeitet wie üblich auf (in Eiswasser eingießen, mit Essigester extrahieren, mit 5 % KHSO₄ waschen, abdestillieren, kristallisieren). Anstelle von Phosphonsäureanhydriden kann man auch Polyphosphorsäureanhydrid (PPA) einsetzen.

Ein weiteres vorteilhaftes Veresterungsverfahren, das auf die gemäß Formel III [R(6) gleich OH] oder in der Liste aufgeführten Carbonsäuren anwendbar ist, ist die direkte Umsetzung von Corticoid-17-carbonsäureestern der Formel II [R(5) gleich OH] mit Hilfe von wasserentziehenden Mitteln, wie Carbodiimiden, vorzugsweise N,N'-Dicyclohexylcarbodiimid (DCCI). Anstelle von DCCi kann man in einigen Fällen auch mit "Molekularsieben" als wasserentziehenden Mitteln arbeiten.

Durch Zusatz einer Säure, z. B. Schwefelsäure, Phosphorsäure, Chlorwasserstoffsäure, Diphenylphosphorsäure, p-Toluolsulfonsäure bzw. von deren Pyridiniumsalzen oder einer organischen Base, z. B. Dimethylaminopyridin (= besonders vorteilhaft in halogenierten Lösungsmitteln, z. B. Methylenchlorid oder in Dimethylformamid) kann die Veresterung katalytisch beschleunigt bzw. optimiert werden, was insbesondere bei sonst schwer reagierenden bzw. empfindlichen Carbonsäuren, z. B. vom Arylessigsäuretyp usw. sehr vorteilhaft ist. Hierbei ist es überraschend, daß die sekundäre 11-Hydroxygruppe in den eingesetzten Corticoid-17-carbonsäureestern (praktisch) in der Regel nicht gleichzeitig mit verestert wird, wie man es öfters bei der Veresterung mit den entsprechenden Säurehalogeniden beobachtet.

In einer besonderen Verfahrensvariante gibt man zu einer Lösung von 1 Moläqu. Corticoid-17-carbonsäureester-21-alkohol [Formel II, R(5) gleich OH] und 1 bis 4 Moläqu. Carbonsäure der Formel III [R(6) gleich OH] vorzugsweise 2 Äquivalente, in absolutem Pyridin eine katalytische Menge Schwefelsäurepyridiniumsalz, sowie nach circa 20 Minuten 1 bis 4 Moläqu. Dicyclohexylcarbodiimid, vorzugsweise 1 bis 2 Moläqu. zu. Man rührt hiernach bei 0 bis 50°C, vorzugsweise 20°C, bis eine DC-Probe keine Ausgangscarbonsäure, sondern nur gewünschte Carbonsäure-21-corticoidester der Formel I anzeigt. Man filtriert vom entstandenen Dicyclohexylharnstoff ab, gießt das Filtrat zweckmäßig in Wasser ein, filtriert (bei Kristallbildung) oder dekantiert (bei öligen bzw. wachsartigen Umfällungen) ab, wäscht mit Wasser nach (gegebenenfalls extrahiert man auch mit Extraktionsmittel, insbesondere Dichlormethan) trocknet, kristallisiert wie üblich um oder stellt, falls erforderlich, die Reaktionsprodukte durch übliche Chromatographie, vorzugsweise an Kieseigel, rein dar.

Anstelle von Pyridin können in einigen Fällen auch andere inerte Lösungsmittel, wie z.B. Tetrahydrofuran, Dioxan, Methylenchlorid, Dimethylformamid zweckmäßig unter Hinzugabe von tertiären Basen, beispielsweise Pyridin, 4-Dimethylaminopyridin verwendet werden. Bei Verwendung von Molekularsieben als wasserentziehenden Mittel sind letztere Lösungsmittel vorzuziehen.

Für die Veresterung mit den empfindlichen Aryl-essigsäuren hat sich weiterhin folgende Variante bewährt: 1 Äqu. Carbonsäure wird bei 0°C in absolutem Dichlormethan gelöst, und nacheinander wird mit 1 Äqu. DCCI, bis 0,2 Äqu. 4-N,N'-Dimethylaminopyridin und einer Lösung von 1 Äqu. Corticosteroid-17-carbonsäureester-21-alkohol in absolutem Dichlormethan versetzt und 18 bis 48 Stunden bei 20°C gerührt. Nach üblicher Aufarbeitung kann der gewünschte Ester der Formel I rein dargestellt werden. Anstatt DCCI kann auch Molekularsieb verwendet werden.

In einer weiteren Veresterungsmethode wird Corticoid-17-carbonsäureester-21-[tert.-Butyidimethylsilyl-(O)-ether] in absolutem Tetrahydrofuran mit 1 Moläqu. Carbonsäure und Trifluoressigsäureanhydrid versetzt, und nach etwa 1 bis 6 Stunden Rühren wird bei 20°C wie üblich aufgearbeitet.

Man kann aber auch direkt die Carbonsäure sowie den Corticoid-17-carbonsäureester-21-alkohol (freie Form) mit Trifluoressigsäureanhydrid zum gewünschten 21-Carbonsäureester umsetzen (= Bildung des gemischten Anhydrids aus Carbonsäure und Trifluoressigsäure, das dann mit dem 21-Alkohol zum 21-Ester reagiert).

### zu Verfahrensvariante b:

Eine weitere vorteilhafte Verfahrensvariante, die zu den erfindungsgemäßen Corticoiden führt, besteht darin, daß man ein Corticoid-17-carbonsäureester-21-halogenid, vorzugsweise 21-lodid oder 21-Bromid oder 21-Sulfonat, vorzugsweise 21-p-Chlorbenzolsulfonsäureester oder 21-methansulfonsäureester mit den Metallsalzen, vorzugsweise Alkalisalzen oder Trialkylammoniumsalzen, der in Liste 2 aufgeführten Carbonsäuren in inerten organischen Lösungsmitteln, vorzugsweise Dimethylsulfoxyd, Dimethylformamid, Butanon-(2), Aceton, Acetonitril, 1 bis 16 Stunden, vorzugsweise 1 bis 10 Stunden bei 20°C bis zu den Siedepunkten der verwendeten Lösungsmittel, vorzugsweise ca. 50°C, erhitzt und nach üblicher Aufarbeitung, vorzugsweise Eingießen von Wasser, Abfiltrieren oder Abdekantieren des Niederschlags und üblicher Reindarstellung isoliert.

Bei dieser nucleophilen Austauschreaktion einer 21-Halogenid- bzw. 21-Sulfonsäureestergruppe gegen eine Carbonsäureestergruppe ist es überraschend, daß unter den vorzugsweise alkalischen Reaktionsbedingungen die für das Wirkungsprofil mitverantwortliche 17-Carbonsäureestergruppe in den Verfahrensprodukten nicht gleichzeitig verseift wird.

Für die gemäß Verfahrensweisen a) und b) hergestellten Verbindungen I gilt, daß eine Hydroxygruppe in 11-Stellung gegebenenfalls nach üblichen Methoden zur Ketogruppe oxydiert werden kann. Vorzugsweise wird diese Oxydation mit Chromtrioxid in saurem Medium und in einem inerten organischen Lösungsmittel durchgeführt. Eine im Corticoidteil vorhandene 9(11)-Doppelbindung kann gegebenenfalls durch Addition von Halogenwasserstoffsäure oder durch Chlor nach üblichen bekannten Methoden in die entsprechenden erfindungsgemäßen Corticoid-17,21-dicarbonsäureester mit einer 11β-Hydroxyl-9α- Halogenidgruppe (9 α F, Cl) oder 11β-9a - Dichlorgruppe überführt werden.

Die Verfahrensprodukte besitzen wertvolle pharmakologische Eigenschaften. Sie sind insbesondere lokal und topisch sehr stark antiphlogistisch wirksam und zeigen teilweise ein überraschend sehr gutes Verhältnis von lokaler zu systemischer antiinflammtorischer Wirkung, das gegenüber analogen Corticoid-17,21-diestern sowie z.B. gegenüber bekannten Corticoid-17-alkylcarbonat-21-estem, die im 21-Esterrest keine Aryl-gruppe tragen, so z. B. 21-Estergruppen mit einer 21-Alkylgruppe, oft deutlich überlegen ist, wie aus pharmakologischen Standardtests hergeleitet werden kann. Demgemäß ist Gegenstand der Erfindung auch ein Mittel zur Behandlung entzündlicher Dermatosen bestehend aus einer Verbindung der Formel I.

Die Verfahrensprodukte können in der Veterinär- und Humantherapie zur Behandlung von entzündlichen Dermatosen verschiedenster Genese in Form von Suspensionen, Salben, Cremes, Sprays usw. Verwendung finden. Dabei ist als besonders vorteilhaft für die lokale und topische Therapieform herauszuheben, daß die Verfahrensprodukte aufgrund ihres äußerst günstigen Verhältnisses von lokaler zu systemischer antiphlogistischer Wirkung auch bei hochdosierter und langanhaltender Therapie praktisch nur geringfügige systemische Nebenwirkungen hervorrufen können. Bei äußerlicher Behandlung werden Salben, Cremes, Suspensionen usw. mit einer Konzentration von 0,01 bis 2 Gew.-% verwendet. Insbesondere zeigen die Verfahrensprodukte in pharmakologischen Tests einen zum Teil wesentlich besseren Split (Verhältnis) von lokaler/systemischer antiinflammatorischer Wirkung als entsprechende Präparate mit einer 21-Estergruppe, die im Esterteil keine Aryl-anteile, wie es bei den erfindungsgemäßen Verbindungen der Fall ist, aufweisen. Weiterhin zeigen die Verfahrensprodukte teilweise auch eine stärkere lokale antiphlogistische Wirksamkeit als die zuletzt genannten Analogpräparate. Darüber hinaus können die erfindungsgemäßen Corticoid-17,21-dicarbonsäureester gegenüber den analogen zuletzt genannten Corticoid-17,21-diestem oft eine noch geringere Haut-Atrophogenität aufweisen, was ein weiterer Vorteil für eine dermatotherapeutische Behandlung ist.

Darüber hinaus können die erfindungsgemäßen Verfahrensprodukte mit diversen gut hautverträglichen lokal wirksamen Antibiotika, z. B. vom Typ des Gentamycins, Neomycins, Erythromycins, Tetracyclins oder der Fusidinsäure und anderen, in galenischen Formulierungen kombiniert werden. Derartige Kombinationen aus den Verfahrensprodukten und den lokalen wirksamen Antibiotika können zur Behandlung von primären bakteriellen oder bakteriell superinfizierten entzündlichen Dermatosen verwendet werden.

### Pharmakologischer Versuchsteil

So zeigten z. B. Prednisolon-17-benzoat-21-phenylessigsäure-ester (I) oder Betamethason-17-benzoat-21-phenylessigsäure-ester (II) eine starke lokale antiphlogistische Wirkung bei einem auffallend günstigen Split zur schwachen systemischen Wirksamkeit, wie aus den unten angeführten pharmakologischen Testergebnissen [Vergleichspräparat Prednicarbat (= Prednisolon-17-ethylcarbonat-21-propionat (US-Patentschrift 4 242 334) und (Merck Index 11, 7717))] hervorgeht:
1. Lokale antiphlogistische Wirkung im Crotonöl-Ohrödem an Ratten nach epikutaner Application
Wir verwendeten die Rattenohr-Methode von Tonelli et al., Endocrinology, 77, 625 (1965): Männliche Wistar-Ratten aus eigener Zucht im Gewicht um 50 g wurden am rechten Ohr mit dem Irritans bzw. Testsubstanz enthaltenden Irritans epikutan behandelt. Das linke Ohr blieb unbehandelt. Zur Entzündungsauslösung diente TPA (12-o-Tetradecanoylphorbol-13-acetat, SIGMA P 8139) in Aceton gelöst, 0,2 mg/ml (davon je 20 µl innen bzw. außen). Die zu prüfenden Kortikoide wurden hierin in den angegebenen Endkonzentrationen gelöst. Kontrollen erhielten nur das TPA-Lösungsmittelgemisch. 4 Stunden nach epikutaner Behandlung wurden die Tiere mit CO₂-getötet. Aus dem rechten (behandelten) und dem linken (unbehandelten) Ohr wurden 8 mm Durchmesser messende Scheiben ausgestanzt und sofort gewogen. Diese Differenz als Parameter für den Grad der Entzündung bei Kontrollen (mg, x ± s) wurde gleich 100 gesetzt. Die antiphlogistische Wirkung wird durch Angabe der ca. 50 %igen Hemmdosis in mg/ml charakterisiert:

| Behandlung | mg/ml | x ± s (mg) | Hemmung in % |
|---|---|---|---|
| Kontrolle | - | 21,2 ± 5,1 | - |
| Verb. I | 0,1 | 5,0 ± 3,1 | 76 |
| | 0,3 | 3,1 ± 2,5 | 85 |
| | 1,0 | 2,0 ± 1,4 | 91 |
| | | | |
| Verb. II | 0,1 | 7,0 ± 3,3 | 67 |
| | 0,3 | 4,9 ± 3,3 | 77 |
| | 1,0 | 1,1 ± 0,9 | 95 |
| | | | |
| Prednicarbat | 0,1 | 5,2 ± 3,3 | 75 |
| | 0,3 | 2,6 ± 2,4 | 88 |

Ergebnis: Die extrapolierte 50 %ige Hemmdosis liegt für Verbindung I, Verbindung II und das Vergleichspräparat bei 0,03 mg/ml.
2 a) Prüfung auf systemische antiphlogistische Wirkung im Test
"Antiphlogistische Wirkung nach subcutaner Gabe: Carrageenan-Pfotenödem an Ratten".
Als Test für die akute systemische antiphlogistische Wirkung wurde das Carrageenan-Pfotenödem an Ratten nach der von Winter et al., Proc. Soc. exp. Biol. (NY), 111, 544 (1962), beschriebenen Methode gewählt. Männliche Sprague-Dawley-Ratten im Gewicht um 120 g erhielten die zu prüfenden Substanzen s.c. (0,2 ml/100 g) in Sesamöl gelöst. 30 min später wurde in die linke Hinterpfote 0,1 ml einer 0,5 % Carrageenan-Lösung injiziert. 6 Stunden später wurde die Schwellungszunahme volumetrisch gemessen. Kontrollen erhielten nur Sesamöl.
Die Pfotenvolumina sind in ml, x ± s, angegeben. Die antiphlogistische Wirkung wird auch hier durch Angabe der ca. 50 %igen Hemmdosis in mg/kg charakterisiert.

| Behandlung | Dosis in mg/kg | Ausgangswert | Volumenzunahme |
|---|---|---|---|
| | s.c. | (ml) | (ml) |
| Kontrolle | - | 1,39 ± 0,09 | 0,58 ± 0,16 |
| Verb. I | 0,3 | 1,40 ± 0,12 | 0,46 ± 0,19 |
| | 3,0 | 1,34 ± 0,06 | 0,38 ± 0,15 |
| | | | |
| Verb. II | 0,3 | 1,42 ± 0,05 | 0,56 ± 0,09 |
| | 3,0 | 1,31 ± 0,09 | 0,45 ± 0,14 |
| | | | |
| Prednicarbat | 0,3 | 1,44 ± 0,08 | 0,36 ± 0,13 |
| | 3,0 | 1,37 ± 0,07 | 0,09"0,08* |

Ergebnis: Die Versuchsauswertung mit dem Dunnett-Test ergab bei beiden Dosierungen der Verbindungen I und II keine signifikante Hemmwirkung, während Prednicarbat mit 3 mg/kg eine signifikante systemische Wirkung hatte (*). Damit sind Verbindungen I und II ca. 10mal geringer wirksam als Prednicarbat, also um diesen Faktor günstiger einzustufen als dieser Standard.
2 b) Prüfung auf systemische Wirkung: Glukoneogenese an Ratten
Eine empflindliche Methode zum Nachweis systemischer Wirkung auf den Kohlehydratstoffwechsel ist die Prüfung der glukoneogenetischen Wirkung von Corticosteroiden an der adrenalektomierten Ratte. Drei Tage vor dem Versuch werden Gruppen zu je 6 Ratten in Pentobarbitalnarkose adrenalektomiert und mit 0,9 % Kochsalzlösung als Trinkflüssigkeit versorgt. Zwei Tage später, d. h. 24 Stunden vor Versuchsbeginn, wird das Futter entzogen, um die Glykogenvorräte in der Leber zu reduzieren.
Am Versuchstag werden die Prüfpräparate subcutan appliziert, gelöst in Sesamöl (2 ml/kg). Sechs Stunden später werden die Tiere dekapitiert, die Leber wird entnommen und 1 g davon in 5 ml 0,6 molarer Perchlorsäure aufgenommen. Nach Homogenisierung wird die freie Glukose im Überstand des Zentrifugats bestimmt, das Zentrifugat (Glykogen) enzymatisch mit Amyloglukosidase aufgespalten und hierin ebenfalls der Glukosegehalt bestimmt (Hexokinase-Methode, Boehringer Mannheim). Es wurden die folgenden Ergebnisse erhalten/Mittelwert" Standardabweichung):

| Behandlung | Dosis | Leberglykogen | Glykogen + Glukose |
|---|---|---|---|
| | (mg/kg s.c.) | | mg/100 g Leber |
| Kontrolle | - | 1,1 ± 0,6 | 11,2 ± 1,7 |
| Verb. I | 0,3 | 2,2 ± 2,1 | 20,4 ± 11,7 n.s. |
| | 3,0 | 43,2 ± 25,8 | 96,0 ± 26,2 |
| | | | |
| Verb. II | 0,3 | 1,1 ± 0,5 | 10,8 ± 1,3 n.s. |
| | 3,0 | 36,1 ± 45,2 | 81,2 ± 61,7 |
| | | | |
| Prednicarbat | 0,3 | 41,2 ± 42,8 | 85,7 ± 40,5* |
| | 3,0 | 93,3 ± 28,9 | 148,2 ± 32,4 |

| | | | |
|---|---|---|---|
| * p<0,05 (t-Test gegen Kontrolle) n.s. - nicht signifikant | | | |

Aus den aufgeführten Ergebnissen zur Glukose-/Glykogenneubildung ist zu entnehmen, daß die Verbindung I und die Verbindung II mit 0,3 mg/kg noch keine signifikante Wirkung haben, während Prednicarbat hier bereits eine geringe aber signifikante (p < 0,05, t-Test) Wirkung aufweist. Ähnlich verhält es sich bei den Dosierungen 3 mg/kg, bei welcher Prednicarbat signifikant stärker ist als Verbindungen I und II. Bei den Verbindungen I und II ist daher der therapeutische Vorteil (geringe systemische Wirkung) größer als bei Prednicarbat.

### Beispiele:

Zu den im folgenden aufgeführten Beispielen sind die nachstehenden allgemeinen Bemerkungen zu machen:

Die Schmelzpunkte werden im Apparat nach Tottoli (Fa. Büchi) oder auf der Kofler-Heizbank der Fa. Reichert (Austria), Typ 7841, bestimmt und sind nicht korrigiert. Die IR-Spektren (in KBr) werden mit dem Gitterspektrophotometer Perkin-Elmer 521 aufgenommen. Es werden jeweils nur die charakteristischen Banden angeführt. Die Aufnahme der UV-Spektren (in Methanol) erfolgte mit dem Spektralphotometer Beckmann DK 1 A. Die massenspektroskopischen Untersuchungen (MS) werden vorwiegend mit dem Gerät MS 9 (Fa. AEI) durchgeführt. Angabe der MS-Spektren (Molgewichtspeak) überwiegend in: MS = m/z = ... (M+H⁺) (Messung mit Reinistopen), d.h. es wurde jeweils die monoisotopische Molmasse erfaßt. In der Regel wurden FAB-MS-Spektren gemessen.
Für die Dünnschicht-Chromatographie (DC) dienten Fertigplatten Kieselgel F₂₅₄ (Fa. Merck). Wenn nicht anders angegeben, wurde als Laufmittel Methylenchorid: Methanol = 19:1 benutzt (Laufstrecke 7 cm). Es wurde jeweils zweimal entwickelt. Die Flecken wurden entweder mit einer UV-Lampe bei 254 nm detektiert oder durch Besprühen mit 10 %-iger methanolischer Schwefelsäure sowie durch Erhitzen auf 100°C sichtbar gemacht. Die R_{F}-Werte sind immer nur relativ zu verstehen. Zur Säulenchromatographie wurde 15 Kieselgel 60, Korngröße 0,063 bis 0,2 mm (Fa. Merck) verwendet.

Bei den Reaktionen mit Carbonsäurechloriden wird in vorteilhafter Weise oft absolutes Dioxan zum Reaktionsgemisch gegeben, z. B. bei substituierten Benzoylchloriden, wobei das Verhältnis Dioxan/Pyridin etwa 1:1 ist, und zur Reaktionsbeschleunigung wird das Reaktionsgemisch oft, insbesondere bei sterisch gehinderten oder weniger reaktiven Carbonsäurechloriden oder -anhydriden auf etwa 60°C erwärmt (DC-Verfolgung der Reaktionsverläufe).

Die Charakterisierung der Reaktionsprodukte kann durch Dünnschicht-Chromatographie (DC) erfolgen; hierbei haben die Reaktionsprodukte R_{F}-Werte von etwa 0,65 bis 0,75. In der Regel werden die Reaktionsprodukte durch Massenspektren mit MS = m/z = ... (M+H⁺) charakterisiert (In der Regel FAB-Spektren); es wird jeweils die monoisotopische Molmasse erfaßt. Die M+H⁺-Werte wurden jeweils aufgerundet. Auch IR-, ¹H-NMR- und UV-Spektren können zur Charakterisierung herangezogen werden.

### Versuch 1 (Erläuterung des Verfahrens)

### Prednisolon-17-n-butyrat-21-(3)-phenylpropionat

Zu einer Lösung von 340 mg Prednisolon-17-n-butyrat in 3 ml absolutem Pyridin wird bei 0°C und unter Rühren eine Lösung von 300 mg 3-Phenylpropionsäurechlorid in 1 ml absolutem Dioxan zugetropft. Nach 5 bis 6 Stunden. Rühren bei 0°C (DC zeigt vollständige Bildung des gewünschten Reaktionsproduktes) gießt man in 100 ml halbgesättigte wäßrige Kochsalzlösung ein, isoliert die Ausfällung (ölig oder Wachs) über ein Faltenfilter, nimmt diese mit Methylenchlorid (oder Essigester) auf, wäscht mit Wasser, trocknet mit Natriumsulfat, destilliert im Vakuum das Lösungsmittel ab, kristallisiert (gegebenenfalls) aus Ethanol/Ether (gegebenenfalls Zusatz von Petrolether) um. Man erhält 400 mg der o. a. Titelverbindung vom Schmp.:
90 bis 93°C (amorph) (aus Petrolether gefällt)
- MS:: m/z = 563 (M+H⁺)
- DC:: R_{F} ~ 0,7

### Versuch 2

### Prednisolon-17-n-butyrat-21-phenylacetat

a) In gleicher Weise, wie unter Versuch 1 beschrieben, werden 350 mg Prednisolon-17-n-butyrat mit 320 mg Phenylacetylchlorid anstelle des 3-Phenylpropionsärechlorids umgesetzt; es wird aufgearbeitet und das Produkt kristallisiert dargestellt. Man erhält 140 mg der o. a. Titelverbindung. Schmp.: ca. 160°C
- MS:: m/z = 549 (M+H⁺)
- DC:: R_{F} = ~ 0,8 (Noch Nebenflecke geringer Intensität im DC oder- und unterhalb des Hauptfleckes bei R_{F} ~ 0,8)

b) Zu einer Lösung von 1,1 g (0,0025 mol) Prednisolon-17-n-butyrat und 1,2 g (0,0088 mol) Phenylessigsäure (5 Stunden in Vakuum über P₂O₅ bei ca. 50 bis 60°C getrocknet) in 6 ml absolutem Pyridin gibt man unter Rühren und bei 20°C eine frisch zubereitete Mischung von 30 mg konzentrierter Schwefelsäure in 2,5 ml absolutem Pyridin (Suspension von Pyridinium-Sulfat). Nach 15 Minuten Rühren gibt man 720 mg (0,0035 mol) N,N'-Dicyclohexylcarbodiimid zu. Aus der anfänglichen klaren Lösung fällt bald ein kristalliner Niederschlag von gebildeten N,N'-Dicyclohexylharnstoff aus. Man rührt solange bis im DC kein Edukt mehr nachweisbar ist und das Reaktionsprodukt bei R_{F} = 0,8 detektierbar ist (in der Regel 16 Stunden Reaktionszeit; eine längere Reaktionszeit, z. B. Stehen bzw. Rühren übers Wochenende beeinträchtigt nicht das Reaktionsergebnis). Hiernach gibt man 0,3 ml Essigsäure oder Essigsäureanhydrid hinzu und läßt den Ansatz noch 1 Stunde bei 20°C 24 bis 48 Stunden im Tiefkühlschrank (ca. -15°C) stehen. Man filtriert vom ausgefallenem N,N'-Dicyclohexylharnstoff ab, wäscht diesen mit etwa - 15°C kaltem Pyridin und rührt das Filtrat in ca. 400 ml viertelgesättigte wäßrige Kochsalzlösung ein, gibt etwa 5 ml Ethanol hinzu, filtriert die öligkristalline Ausfällung ab, wäscht diese mehrmals mit Wasser und nimmt diese mit etwa 20 ml Methylenchlorid auf. Nach dem Trocknen mit Natriumsulfat destilliert man ab und kristallisiert den Rückstand durch Zugabe von Diethylether oder Diisopropylether. Man erhält 1,1 g Prednisolon-17-n-butyrat-21-phenylacetat vom Schmp. ca. 160°C, das aus tert.-Butanol/Diethylether umkristallisiert wird.
- Schmp.:: 164 bis 166°C
- MS:: m/z = 549 (M+H⁺)
- DC:: R_{F} ~ 0,80 (R_{F} v. ED ~ 0,45) keine sichtbaren Nebenflecken oberhalb und unterhalb R_{F} ~ 0,8.

c) Es wird ein weiterer analoger Ansatz, wie unter Versuch 2 b) beschrieben durchgeführt; allerdings wird der saure Katalysator, könzentrierte Schwefelsäure in Pyridin, weggelassen. Nach etwa 5-facher Reaktionszeit, wie unter Versuch 2 b) angegeben, zeigt eine DC-Probe kein Edukt mehr. Nach analoger Aufarbeitung und Reindarstellung, wie unter Versuch 2 b) angegeben, werden 1,0 g Prednisolon-17-n-butyrat-21-phenylacetat mit den gleichen Kenndaten, wie unter Versuch 2 b) angegeben, erhalten.

Wird anstelle von Pyridin absolutes Dimethylformamid als Lösungsmittel verwendet, so erhält man die Titelverbindung ebenfalls mit den gleichen Daten.

d) Es wird ein weiterer analoger Ansatz, wie unter Versuch 2 b) beschrieben durchgeführt. Ansteile der Schwefelsäure werden aber 60 mg p-Toluolsulfonsäure zugegeben. Nach analoger Aufarbeitung und Reindarsteliung, wie unter Versuch 2 b) angegeben, werden 1,3 g Prednisolon-17-n-butyrat-21-phenylacetat mit den gleichen Kenndaten, wie in Versuch 2 b) angegeben, erhalten.

e) Zu einer Lösung von 2,16 g Prednisolon-17-n-butyrat und 1,22 g Phenylessigsäure in 40 ml absolutem Methylenchlorid gibt man bei 0°C und Rühren 120 mg 4-Dimethylaminopyridin und 1,75 g Dicyclohexylcarbodiimid. Die zunächst klare Reaktionslösung trübt sich bald. Nach ca. 36 Stunden Rühren bei Zimmertemperatur zeigt eine DC-Probe kein Edukt mehr. Man bewahrt dann 2 Tage bei -15°C (Tiefkühlschrank) auf, filtriert den ausgefallenen Dicyclohexylharnstoff ab, wäscht diesen mit etwa -15°C kaltem Methylenchlorid und zieht das organische Lösungsmittel im Vakuum ab. Der hinterbliebene Rückstand wird aus siedendem Diethylether zur Kristallisation gebraucht und aus Ethanol/Diethylether umkristallisiert. Man erhält 1,9 g der o. a. Titelverbindung (strahlend weiße Kristalle) mit den gleichen unter Versuch 2 b) angegebenen Daten (MS, DC, Schmelzpunkt). Der Schmelzpunkt ist gegenüber Versuch 2 b) um etwa 2° höher: Schmp.: 166 bis 168°C,

f) bei einem analogen Ansatz gemäß e) wird das Methylenchlorid durch Dimethylformamid als Lösungsmittel ersetzt. Ansonsten wird genau wie unter Versuch 2 e) angegeben verfahren. Nach der Aufarbeitung erhält man 1,7 g der o. a. Titelverbindung mit Schmp.: 165 bis 167°C

### Beispiel 1

### Prednisolon-17-benzoat-21-phenylacetat

Werden, wie in Versuch 2 b) beschrieben, 1,1 g Prednisolon-17-benzoat mit 1,2 g Phenylessigsäure und 720 mg N,N'-Dicyclohexylcarbodiimid sowie Pyridiniumsulfat in 8 ml absolutem Pyridin umgesetzt, aufgearbeitet und rein dargestellt, so erhält man nach Kristallisation mit Diisopropylether 850 mg der o. a. Titelverbindung vom Schmp.: 106°C
- MS:: m/z = 583 (M+H⁺)
- DC:: R_{F} ~ 0,8

### Beispiel 2

### Prednisolon-17,21-bis-[phenylacetat]

Werden, wie in Versuch 2 b) beschrieben, 1,1 g Prednisolon-17-phenylacetat mit 1,2 g Phenylessigsäure und 730 mg N,N'-Dicyclohexylcarbodiimid sowie Pyridiniumsulfat in 7,5 ml absolutem Pyridin umgesetzt, aufgearbeitet und rein dargestellt, so erhält man nach Digerieren mit Petrolether 1,0 g amorphes Produkt, das o. a. Titelverbindung darstellt.
- MS:: m/z = 597 (M+H⁺)
- DC:: R_{F} ~ 0,8

### Versuch 3

### Betamethason-17-n-valerat-21-phenylacetat

a) In gleicher Weise, wie in Versuch 2 b) beschrieben, werden 2,4 g Betamethason-17-valerat mit 2,4 g Phenylessigsäure, Pyridiniumsulfat (69 mg konz. Schwefelsäure in 2 ml Pyridin) sowie 1,44 g N,N'-Dicyclohexylcarbodiimid in 12 ml absolutem Pyridin 72 Stunden bei 20°C umgesetzt, aufgearbeitet und rein dargestellt. Nach Kristallisation der ursprünglich wachsartigen Ausfällung aus Diethylether erhält man 1,6 g der obigen Titelverbindung. Schmp.: 178 bis 181°C
- MS:: m/z = 595 (M+H⁺)
- DC:: R_{F} ~ 0,75

b) In gleicher Weise, wie in Versuch 2 e) beschrieben, werden 12 g Betamethason-17-valerat in 200 ml Methylenchlorid mit 6,1 g Phenylessigsäure, 8,75 g N,N'-Dicylohexylcarbodiimid sowie 600 mg 4-Dimethylaminopyrimidin 16 Stunden bei 0°C umgesetzt, aufgearbeitet und rein dargestellt. Durch Kristallisation aus Diethylether und zweimalige Umkristallisation aus Ethanol/Methylenchlorid + Diethylether erhält man 6,2 g der o. a. Titelverbindung mit den gleicher unter a) angegebenen Daten. Schmp. 178 - 179°C.

Wurde aber analog 24 Stunden bei Zimmertemperatur (22°C) umgesetzt, so erhielt man als Hauptprodukt 8,2 g Betamethason-17n-valerat-11,21-bis-phenylacetat (Kristallisation aus Ethanol). Schmp. 121°C;
- MS:: m/z = 713 (M+H⁺)
- DC:: R_{F} ~ 0,85-0,90

Aus der Mutterlauge erhält man nach Kristallisation aus Diethylether 2,8 g die o.g. Titelverbindung mit den gleichen Daten wie unter Versuch 3a).

### Versuch 4

### Prednisolon-17-n-butyrat-21-phenylacetat

a) Zu einer Lösung von 2,10 g Prednisolon-17-n-butyrat und 1,20 g Phenylessigsäure in 40 ml absolutem Methylenchlorid gibt man bei 0°C und Rühren 120 mg 4-Dimethylaminopyridin und 1,75 g Dicyclohexylcarbodiimid. Die zunächst klare Reaktionslösung trübt sich bald. Nach ca. 36 Stunden Rühren bei Zimmertemperatur zeigt eine DC-Probe kein Edukt mehr. Man bewahrt dann 2 Tage bei -15°C (Tiefkühlschrank) auf, filtriert den ausgefallenen Dicyclohexylharnstoff ab, wäscht diesen mit etwas -15°C kaltem Methylenchlorid und zieht das organische Lösungsmittel im Vakuum ab. Der hinterbliebene Rückstand wird aus siedendem Diethylether zur Kristallisation gebracht und aus Ethanol/Diethylether umkristallisiert. Man erhält 1,8 g der o. a. Titelverbindung mit den gleichen unter Versuch 2b) angegebenen Daten (MS, DC, Schmelzpunkt. Der Schmelzpunkt ist gegenüber Versuch 2 b) um etwa 3° höher: Schmp.: 167 bis 169°C,

b) bei einem analogen Ansatz gemäß Versuch 4 a) wird das Methylenchlorid durch Dimethylformamid als Lösungsmittel ersetzt. Ansonsten wird genau wie unter Versuch 4 a) angegeben, verfahren. Nach der Aufarbeitung erhält man 1,7 g der o.a. Titelverbindung mit Schmp.: 166°C

### Versuch 5

### Prednisolon-17-n-butyrat-21-phenylacetat

a) Eine Mischung von 216 mg Prednisolon-17-n-butyrat oder 270 mg 21-(tert.-Butyldimethylsiloxy-prednisolon-17-n-butyrat, 136 mg Phenylessigsäure, 210 mg Trifluoressigsäureanhydrid sowie 6 mg wasserfreie p-Toluolsulfonsäure wird 7 Stunden in 40 ml absolutem Toluol oder Benzol am Rückfluß gekocht. Hiernach wird in 6 %ige wäßrige Natriumbicarbonatlösung eingegossen und intensiv durchgerührt. Man wäscht mit Wasser, trocknet, zieht das Lösungsmittel ab und chromatographiert an Kieselgel (s. Versuch 2 b)). Das bei DC = R_{F} ~ 0,7 laufende Produkt wird aus Diethylether kristallisiert. Es ist in allen Daten mit dem unter Versuch 2 angegebenen Reaktionsprodukt identisch.

b) Bei einem weiteren Ansatz werden 700 mg Prednisolon-17-n-butyrat in 20 ml absolutem Dioxan mit 1,5 g Phenylessigsäure und 0,75 ml Trifluoressigsäureanhydrid versetzt. Nach 30 Stunden Rühren bei 20C rührte man in 40 ml Wasser, das 2 g Natriumbicarbonat enthält, ein. Das erhaltene wachsartige Produkt wird nach dem Trocknen wie unter Versuch 2 b) chromatographiert und aus Diethylether kristallisiert. Man erhält o. a. Titelverbindung mit den gleichen, wie unter Versuch 2 b), angegebenen Daten.

### Versuch 6

### Prednisolon-17-n-butyrat-21-phenylacetat

150 mg Phenylessigsäure und 430 mg Prednisolon-17-n-butyrat werden in 3 ml abs. Methylenchlorid sowie 5 ml absolutem Pyridin gelöst und mit 0,25 ml 50 %iger Propanphosphonsäureanhydridlösung in absolutem Methylenchlorid sowie 10 mg 4-Dimethylaminopyridin versetzt. Nach 8 Stunden Rühren bei ca. 40°C (Ölbad) wird in Eiswasser, das zur Neutralisation Natriumbicarbonat enthält, eingegossen. Es wird mit Essigester extrahiert, mit wäßriger KHSO₄-Lösung sowie Wasser gewaschen. Nach dem Abdestillieren wird der Rückstand an Kieselgel chromatographiert. Neben Ausgangsmaterial und Prednisolon enthält eine Eluatsfraktion auch die gewünschte o. a. Titelverbindung mit den gleichen Daten, wie unter Versuch 2 b) angegeben.

### Versuch 7

### Prednisolon-17-n-butyrat-21-phenylacetat

Zu einer Lösung von 220 mg Prednisolon-17-n-butyrat in 2 ml absolutem Pyridin wird bei 0°C und unter Rühren eine Lösung von 400 ml Phenylessigsäureanhydrid in 1 ml absolutem Dioxan zugetropft. Nach 5 bis 6 Stunden Rühren bei 0°C und 16 Stunden Rühren bei 20°C gießt man in 100 ml halbgesättigte wäßrige Kochsalzlösung ein, isoliert die wachsartige Ausfällung über ein Faltenfilter, nimmt diese mit Methylenchlorid (oder Essigester) auf, wäscht mit Wasser, trocknet mit Natriumsulfat, destilliert im Vakuum das Lösungsmittel ab, kristallisiert mit Diisopropylether oder Diethylether oder Petrolether, filtriert ab und kristallisiert aus Ethanol/Ether (gegebenenfalls Zusatz von Petrolether) um. Man erhält 135 mg der o. a. Titelverbindung vom Schmp.: 165°C
- MS:: m/z = 549 (M+H*)
- DC:: R_{F} ~ 0,7

### Versuch 8

### Prednisolon-17-n-butyrat-21-phenylacetat

a) Eine Lösung von 500 mg Prednisolon-17-n-butyrat-21-mesylat (oder eine äquimolare Menge des analogen -21-p-chlor-benzolsulfonats), 145 mg Phenylessigsäure und 112 mg Triethylamin (hierbei findet intermediär Bildung des Triethylammoniumphenylacetats statt) in 25 ml Dimethylformamid (oder Acetonitril) wird 3 Stunden bei ca. 45°C (Ölbad) gerührt. Hiernach wird das Dimethylformamid bzw. Acetonitril im Vakuum abdestilliert und der Rückstand mit 30 ml Methylenchlorid behandelt. Man wäscht die organische Phase hintereinander mit 1 N wäßriger Salzsäure und 4 mal mit Wasser. Nach Chromatographie und Kristallisation aus Diethylether erhält man die o.a. Titelverbindung mit den gleichen Daten, wie unter Versuch 2 b) angegeben.

b) Zur gleichen Titelverbindung gelangt man, wenn man 600 mg Prednisolon-17-n-butyrat-21-desoxy-21-iodid, 150 mg Phenylessigsäure, 2,5 ml Triethylamin in 25 ml Acetonitril 45 Minuten am Rückfluß kocht und wie unter a) aufarbeitet und isoliert.

c) 600 mg Prednisolon-17-n-butyrat-21-desoxy-21-iodid werden mit 200 ml Kaliumphenylacetat (Rhone-Poulenc) in 25 ml absolutem Dimethylformamid 40 Minuten auf 100°C (Ölbad) unter Rühren erhitzt. Hiernach kühlt man ab und gießt in halbgesättigte wäßrige Kochsalzlösung ein, wobei ein abfiltrierbares öliges Wachs ausfällt, das nach dem Abfiltrieren, Waschen mit Wasser und Trocknen (Vakuum über P₂O₅) an Kieselgel chromatographiert wird und nach dem Kristallisieren die o. a. Titelverbindung mit den gleichen Daten wie in Versuch 2 a) bzw. 2 b) ergibt.

### Versuch 9

### Prednisolon-17-benzoat-21-zimtsäureester

In gleicher Weise wie unter Versuch 1 beschrieben, werden 350 mg Prednisolon-17-benzoat anstelle des Prednisolon-17-n-butyrats mit 320 mg Zimtsäurechlorid in Pyridin/Dioxan bei 0°C umgesetzt. Nach analoger Aufarbeitung und Isolierung erhält man durch Fällen mit Petrolether die o.a. Titelverbindung in amorpher Form.
- MS:: = 595 (M+H⁺)
- DC:: R_{F} ~ 0,8

### Versuch 10

### Prednisolon-17-benzoat-21-p-methoxyzimtsäureester

Werden in Versuch 9 anstelle des Zimtsäurechlorids 380 mg p-Methoxyzimtsäurechlorid eingesetzt, so erhält man nach analoger Reaktionsführung, Aufarbeitung und Isolierung durch Fällen mit Petrolether die o.a. Titelverbindung (amorph).
- MS:: = 625 (M+H⁺)
- DC:: R_{F} ~ 0,8

### Versuch 11

### Betamethason-17-benzoat-21-zimtsäureester

In gleicher Weise wie unter Versuch 1 beschrieben, werden 360 mg Betamethason-17-benzoat anstelle des Prednisolon-17-n-butyrats mit 320 mg Zimtsäurechlorid in Pyridin/Dioxan bei 0°C umgesetzt. Nach analoger Aufarbeitung und Isolierung erhält man durch Fällen mit Petrolether die o.a. Titelverbindung (amorph).
MS:- = 628 (M+H⁺)
DC: R_{F} ~ 0,8

### Versuch 12

### Betamethason-17-benzoat-21-p-methoxyzimtsäureester

Werden in Versuch 11 anstelle des Zimtsäurechlorids 380 mg p-Methoxyzimtsäurechlorid eingesetzt, so erhält man nach analoger Reaktionsführung, Aufarbeitung und Isolierung durch Fällen mit Petrolether die o.a. Titelverbindung in amorpher Form.
- MS:: = 658 (M+H⁺)
- DC:: R_{F} ~ 0,8

### Beispiel 3

### Betamethason-17-benzoat-21-phenylacetat

Zu einer Lösung von 1,37 g Betamethason-17-benzoat und 1,32 g Phenylessigsäure (getrocknet) in 6 ml absol. Pyridin gibt man unter Rühren und bei 20°C Pyridiniumsulfat (aus 50 mg konz. Schwefelsäure in 1,7 ml absol. Pyridin gemäß Versuch 2 b) hergestellt). Nach 30 Minuten Rühren (20°C) gibt man 790 mg N,N'-Dicyclohexylcarbodiimid hinzu. Nach 60 Std. Rühren bei 20°C zeigt das DC vollständige Umsetzung zur o.a. Titelverbindung. Nach Zugabe von 0,25 ml Essigsäureanhydrid wird 24 Stunden im Tiefkühlschrank (-15°C) aufbewahrt. Man filtriert den ausgefallenen Dicyclohexylharnstoff ab, wäscht ihn mit 10 ml absol. Pyridin (-15°C) nach und engt das Filtrat an der Hochvakuumpumpe ein. Man erhält 1,75 g Wachs, das an Kieselgel (Merck AG, 35-70 my) (Säulenfüllung: 24 cm Höhe, 3,5 cm Breite) mit ca. 1l säurefreiem Methylenchlorid + 0,5 % Zusatz an Methanol chromatographiert wird. Nach Abdestillieren des Eluationsmittels erhält man nach Anreiben und Kristallisieren mit Diisopropylether 820 mg der o.a. Titelverbindung in DC-reinster Form. Schmp. 186°C.
- MS:: = 616; (M+H⁺)
- DC:: R_{F} ~ 0,85

### Liste

A) Folgende Carbonsäuren der Formel III bzw. deren aktivierte Derivate, kommen als Ausgangssubstanzen beispielsweise in Frage:
1. Ein- oder mehrfach substituierte Benzoesäuren der Formel 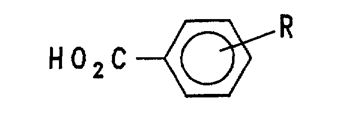 R = (ein- oder mehrfach) substituiertes Alkoxy, Methylendioxy, Acylamino, Dialkylamino, Fluor, Chlor, Mercaptoalkyl, Phenoxy, Alkyl, Dialkylamino, Amino:
   2-, 3- oder 4-Methoxy-benzoesäure; 2-, 3- oder 4-Chlor-benzoesäure; Fluorbenzoesäure; 2,4-, 3,4- oder 2,6-Difluor- oder dichlor-benzoesäure; 2-, 3- oder 4-Methyl-benzoesäure; 3,5-Dimethylbenzoesäure; 4-(t-Butoxy)-benzoesäure; 4-t-Butyl-benzoesäure; 3,4-Methylendioxy-benzoesäure; 2,3-, 3,5- oder 2,6-Dimethoxybenzoesäure; 2,3,4-Trimethoxybenzoesäure; 4-Amino-benzoesäure (PABA),
2. Aryl-essigsäuren und Analoge bzw. Homologe Phenylessigsäure; 2-Methyl- oder 3-Methyl- oder 4-Methyl-phenylessigsäure, 4-(t-Butyl)-phenylessigsäure; 2-Chlor- oder 3-Chlor- oder 4-Chlor-phenylessigsäure; 2,6-Dichlor- oder 3,4-Dichlorphenylessigsäure; 2-Fluor- oder 3-Fluor- oder 4-Fluorphenylessigsäure; 2,6-Difluor-phenylessigsäure; 2-Nitro- oder 3-Nitro- oder 4-Nitrophenylessigsäure; 2,4-Dinitro-phenylessigsäure; 2-Methoxy- oder 3-Methoxy- oder 4-Methoxy-phenylessigsäure; 4-Benzyloxy-phenylessigsäure; 3-Chlor-4-methoxy-phenylessigsäure; 3-Brom-4-methoxy-phenylessigsäure; 3-Nitro-4-methoxy-phenylessigsäure; 3,4-Dimethoxy-phenylessigsäure; 2,3,4-Trimethoxyphenylessigsäure; 3,4-Methylendioxy-phenylessigsäure; 3,4-Diethoxyphenylessigsäure;
   4-Benzyloxy-phenylessigsäure; 4-(2-Methoxybenzyloxy)-phenylessigsäure; 4-(4-Fluorbenzyloxy)-phenylessigsäuren; 3-Phenyl-propionsäure; D,L-2-Phenylpropionsäure; 3-[4-Methyl-phenyl]-propionsäure, 3-[4-Chlor- oder 4-Fluor- oder 4-Methoxy-phenyl]-propionsäuren; (S)-(+)-2-Phenylpropionsäure; (R)-(-)-2-Phenylpropionsäure; 4-Phenyl-buttersäure; D,L- oder (S)- oder (R)-2-(4-Isobutylphenyl)-propionsäure (Ibuprofen); 4-(Isobutylphenyl)-essigsäure (Ibufenac); (4-Phenyl)buttersäure.

## Patentansprüche

1. Corticoid-17,21-dicarbonsäureester der Formel I 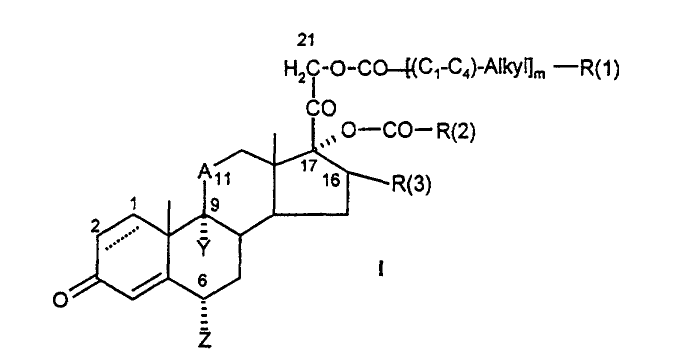 in welcher bedeuten:
A CHOH und CHCI in beliebiger sterischer Anordnung, (CH₂), C=O, 9(11)-Doppelbindung;
Y Wasserstoff, Fluor, Chlor;
Z Wasserstoff, Fluor, Methyl;
R(1) Phenyl,
das unsubstituiert ist oder substituiert mit Methylendioxy, Halogen,
Alkyl, Alkoxy, NO₂, NH₂ oder CN;
[(C₁-C₄)-Alkyl]
gesättigt, geradkettig oder durch weitere Alkylgruppen verzweigt;
m 1;
R(2) Phenyl oder Benzyl;
R(3) Wasserstoff oder α- oder ß-Methyl.

2. Verbindung nach Anspruch 1, **dadurch gekennzeichnet, dass** es sich um Prednisolon-17-benzoat-21-phenylacetat oder um Betamethason-17-benzoat-21-phenylacetat handelt.

3. Verfahren zum Herstellen einer Verbindung I nach Anspruch 1, **dadurch gekennzeichnet, daß** man
a) eine Verbindung der Formel II, 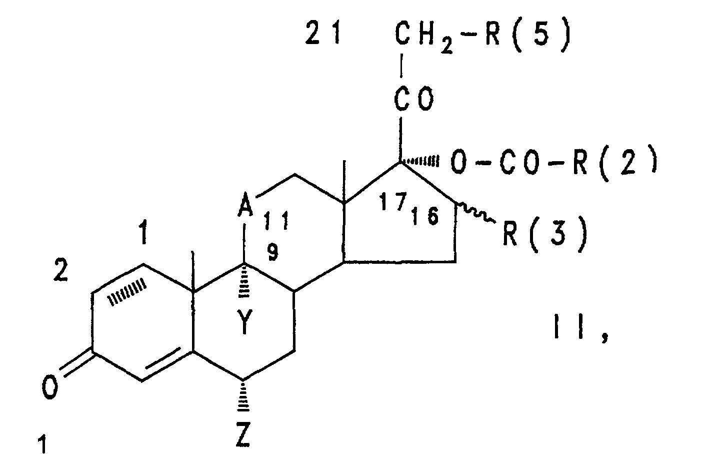 in der R(5) gleich OH ist und die übrigen Substituenten die in Anspruch 1 angegebenen Bedeutungen haben,
a 1) mit einer aktivierten Carbonsäure der Formel III, vorzugsweise einem Halogenid oder Anhydrid oder Azolid,
R(6)-CO-[(C₁-C₄)-Alkyl]ₘ-R(1) III
umsetzt, wobei bedeuten:
m 1, und
[(C₁-C₄)-Alkyl] und R(1) die in Anspruch 1 angegebenen Bedeutungen haben und
R(6) Cl, Br, O[-CO―[(C₁-C₄)-Alkyl]ₘ-R(1)]₁-, -O-C(O)-CF₃ oder ein anderes _ aktiviertes Säureradikal, oder
a 2) mit einer Carbonsäure der Formel III selbst, in der
R(6) OH ist,
und die weiteren Substituenten bei Formel III angegeben sind,
in Gegenwart Wasser abspaltender Reagentien (DCCI etc.) umsetzt
oder daß man
b) Verbindungen der Formel II, 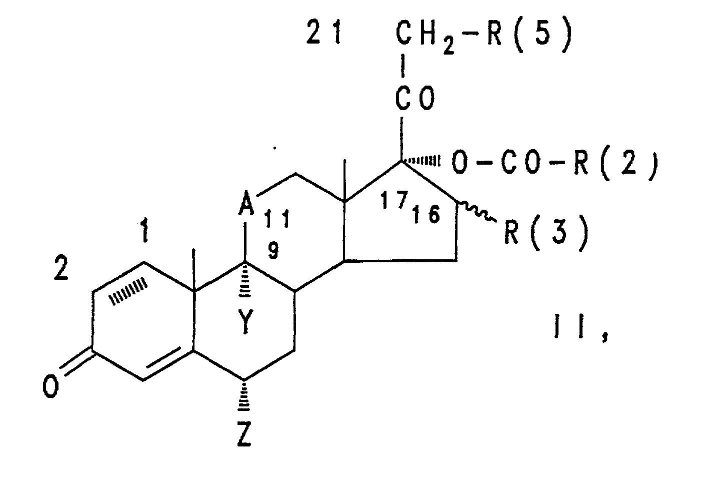 in der R(5) = Br, J, eine Sulfonsäurearyl- oder -alkylestergruppierung ist und die weiteren Substituenten die bei Formel I angegebenen Bedeutung haben, mit einem Salz, vorzugsweise K- oder Na-Salz oder einem Trialkylammoniumsalz, einer Carbonsäure der Formel III,
R(6)-CO- [(C₁-C₄)-Alkyl]ₘ-R(1) III
in der
R(6) -[O⁻Me⁺] bedeutet,
und die weiteren Substituenten die bei Formel III angegebenen Bedeutungen haben, umsetzt,
wobei Me vorzugsweise das Kation eines Alkalisalzes oder eines Trialkylammoniumsalzes ist.

4. Medikament, **gekennzeichnet durch** einen wirksamen Gehalt einer Verbindung I nach Anspruch 1.

5. Verwendung einer Verbindung I nach Anspruch 1 zum Herstellen eines Medikaments zur Behandlung von Dermatosen.

## Claims

1. A corticoid 17,21-dicarboxylic ester of the formula I 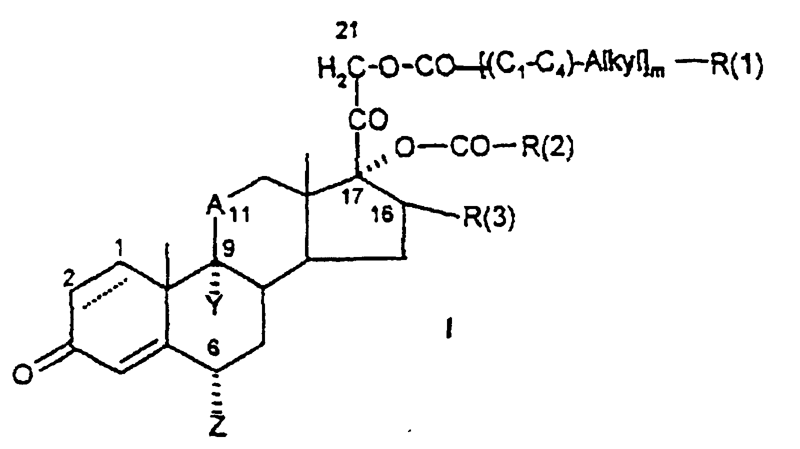 in which:
A is CHOH and CHCl in arbitrary steric arrangement, (CH₂), C=O or 9(11) double bond;
Y is hydrogen, fluorine or chlorine;
Z is hydrogen, fluorine or methyl;
R(1) is phenyl which is unsubstituted or is substituted by methylenedioxy, halogen, alkyl, alkoxy, NO₂, NH₂ or CN;
[(C₁-C₄)-alkyl]
saturated, straight-chain or branched by further alkyl groups,
m is 1;
R(2) is phenyl or benzyl;
R(3) is hydrogen or α- or β-methyl.

2. A compound as claimed in claim 1 which is prednisolone 17-benzoate 21-phenylacetate or betamethasone 17-benzoate 21-phenylacetate.

3. A process for preparing a compound I as claimed in claim 1, wherein
a) a compound of the formula II 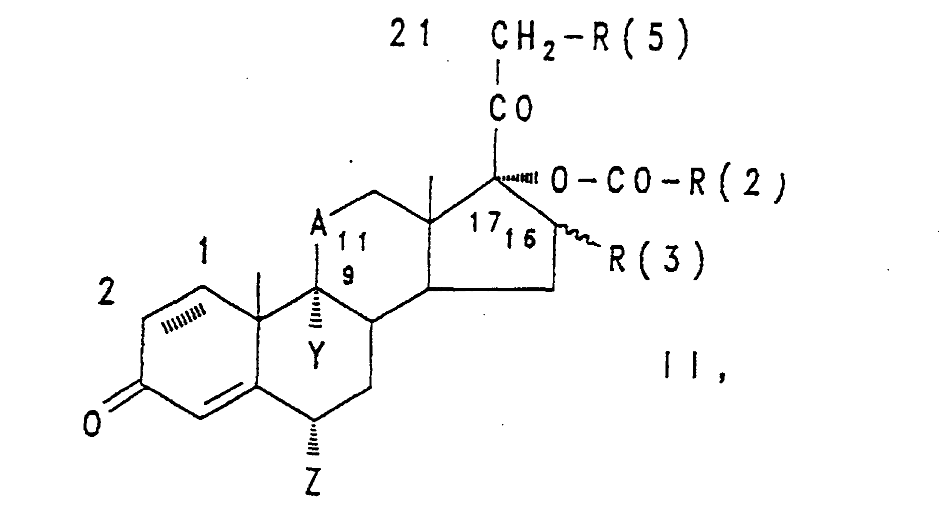 in which R(5) is OH and the remaining substituents have the meanings given in claim 1,
a1) is reacted with an activated carboxylic acid of the formula III, preferably a halide or anhydride or azolide,
R(6)-CO-[(C₁-C₄)-alkyl]ₘ-R(1) III
in which:
m is 1, and
[(C₁-C₄)-alkyl] and R(1) have the meanings given in claim 1, and
R(6) is Cl, Br, O[-CO-[(C₁-C₄)-alkyl]ₘ-R(1)]₁-, -O-C(O)-CF₃, or another activated acid radical, or
a2) is reacted with a carboxylic acid of the formula III itself, in which R(6) is OH,
and the other substituents are given in formula III,
in the presence of water-eliminating reagents (DCCI, etc.),
or wherein
b) compounds of the formula II 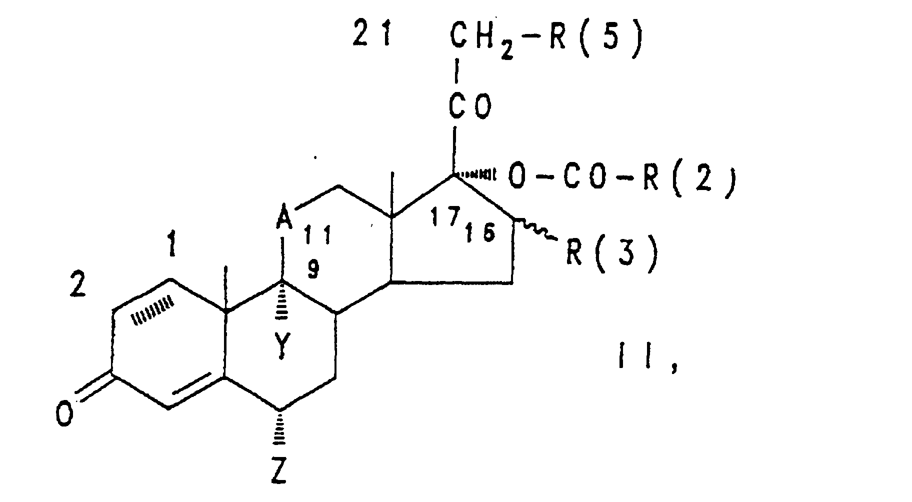 in which R(5) is Br, I, or a sulfonic aryl ester group or sulfonic alkyl ester group, and the other substituents have the meaning given in formula I, are reacted with a salt, preferably a K or Na salt or a trialkylammonium salt, of a carboxylic acid of the formula III,
R(6)-CO-[(C₁-C₄)-alkyl]ₘ-R(1) III
in which
R(6) is -[O⁻Me⁺],
and the other substituents have the meanings given in formula III,
Me preferably being the cation of an alkali metal salt or of a trialkylammonium salt.

4. A pharmaceutical which has an effective content of a compound I as claimed in claim 1.

5. Use of a compound I as claimed in claim 1 for preparing a pharmaceutical for treating dermatoses.

## Revendications

1. 17,21-dicarboxylates de corticoïdes de formule I 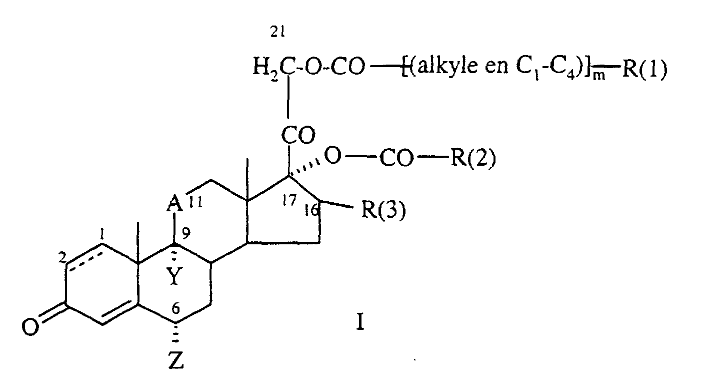 où:
A représente CHOH ou CHCl dans une configuration stérique quelconque, (CH₂), C=O, une double liaison 9(11) ;
Y représente l'hydrogène, le fluor, le chlore ;
z représente l'hydrogène, le fluor, méthyle ;
R(1) représente phényle qui est non substitué ou substitué par méthylènedioxy, halogène, alkyle, alcoxy, NO₂, NH₂ ou CN ;
[alkyle en C₁-C₄] saturé, linéaire ou ramifié par d'autres groupes alkyle ;
m représente 1 ;
R(2) représente phényle ou benzyle ;
R(3) représente l'hydrogène ou α- ou β-méthyle.

2. Composé selon la revendication 1, **caractérisé en ce qu'**il s'agit du 17-benzoate-21-phénylacétate de prednisolone ou du 17-benzoate-21-phénylacétate de bétaméthasone.

3. Procédé de préparation d'un composé I selon la revendication 1, **caractérisé en ce que**
a) on fait réagir un composé de formule II 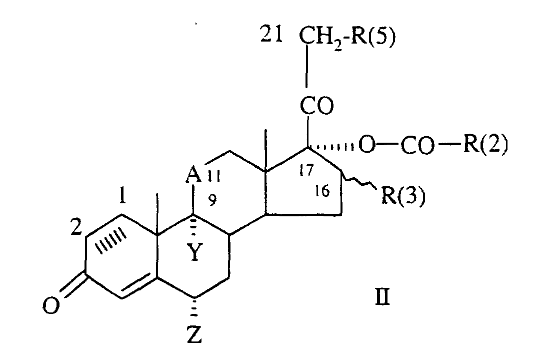 où R(5) est OH et les autres substituants ont les significations indiquées dans la revendication 1,
a1) avec un acide carboxylique activé de formule III, de préférence un halogénure ou un anhydride ou un azolide
R(6)-CO-[(alkyle en C₁-C₄)]ₘ-R(1) III
où:
m représente 1 et
[alkyle en C₁-C₄] et R(1) ont les significations indiquées dans la revendication 1 et
R(6) représente Cl, Br, O[-CO-[alkyle en C₁-C₄]ₘ-R(1)]₁, -O-C(O)-CF₃ ou un autre radical acide activé, ou
a2) avec un acide carboxylique de formule III proprement dit, où
R(6) est OH,
et les autres substituants sont indiqués au sujet de la formule III,
en présence de réactifs clivant l'eau (DCCI, etc.)
ou **en ce que**
b) on fait réagir des composés de formule II 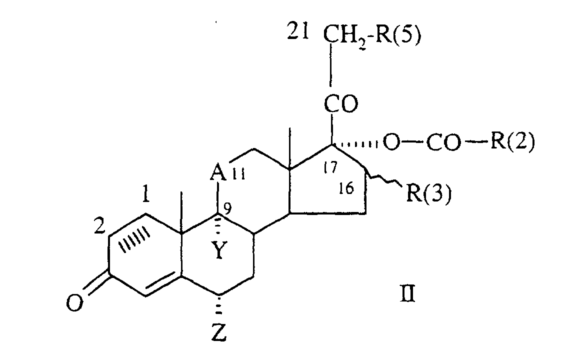 où R(5) = Br, I, un groupement aryl- ou alkylester d'acide sulfonique et les autres substituants ont la signification indiquée au sujet de la formule I, avec un sel, de préférence un sel de K ou de Na ou un sel de trialkylammonium, d'un acide carboxylique de formule III
R(6)-CO-[(alkyle en C₁-C₄)]ₘ-R(1) III
où
R(6) signifie -[O⁻Me⁺]
et les autres substituants ont les significations indiquées au sujet de la formule III, où Me est de préférence le cation d'un sel alcalin ou d'un sel de trialkylammonium.

4. Médicament **caractérisé par** une teneur efficace en un composé I selon la revendication 1.

5. Utilisation d'un composé I selon la revendication 1 pour la préparation d'un médicament pour le traitement des dermatoses.
